# EUROPEAN PATENT APPLICATION

(11) **EP 4 567 105 A1**
(43) Date of publication of application: **11.06.2025**
(21) Application number: 23850170.4
(22) Date of filing: 04.08.2023
(51) Int. Cl.: C12N 5/10, C12N 15/12

(54) **METHOD FOR PRODUCING CARDIAC MUSCLES**

(30) Priority: 05.08.2022 JP 2022125789
(71) Applicant: Kyoto University, Kyoto-shi, Kyoto 606-8501 (JP); Takeda Pharmaceutical Company Limited, Osaka-shi, Osaka 541-0045 (JP)
(72) Inventor: YOSHIDA, Yoshinori, Kyoto-shi, Kyoto 606-8501 (JP); KOAKUTSU, Misato, Kyoto-shi, Kyoto 606-8501 (JP); MIKI, Kenji, Kyoto-shi, Kyoto 606-8501 (JP)
(74) Representative: Jones, Nicholas Andrew
(86) International application number: PCT/JP2023/028543
(87) International publication number: WO 2024/029617

(57) **Abstract**

The present invention provides a method for producing atrial myocytes from early cardiomyocytes, including (a) a step of culturing a cell population containing early cardiomyocytes in the presence of a Notch signal inhibitor. The present invention also provides a cell population containing atrial myocytes, which is obtained by the method of the present invention. The present invention provides use of a Notch signal inhibitor in the production of an atrial myocyte from an early cardiomyocyte.

## Description

### [Technical Field]

The present invention relates to a method for producing atrial myocytes from early cardiomyocytes with high efficiency and a cell population containing atrial myocytes obtained by the method.

### (Background of the Invention)

Techniques for inducing differentiation of stem cells, such as induced pluripotent stem cells (iPSCs) and embryonic stem cells (ESCs), into cardiomyocytes are expected to be applied to regenerative medicine for cardiac diseases and drug discovery using cardiac disease model experimental systems. Cardiomyocytes include various subtypes of cells, such as ventricular muscle, atrial muscle, and pacemaker cells, each of which has a different function. At present, cell populations containing cardiomyocytes, which are induced to differentiate from stem cells, contain a mixture of multiple subtypes of cardiomyocytes and may also contain non-cardiomyocytes such as undifferentiated cells. Therefore, technological developments are ongoing with the aim of selectively obtaining specific subtypes of cells according to the purpose of application.

Patent Literature 1 discloses a method for obtaining a cell population enriched in ventricular myocyte or atrial myocytes by using the expression level of CD151 as an index. However, such conventional techniques do not disclose a technique for selectively obtaining atrial myocytes from early cardiomyocytes.

### [Citation List]

### [Patent Literature]

[Patent Literature 1]
WO WO2021/033699

### [Summary of Invention]

### [Technical Problem]

The present invention aims to provide a method for producing atrial myocytes from early cardiomyocytes with high efficiency. The present invention further aims to provide a cell population predominantly containing atrial myocytes produced by the aforementioned method.

### [Solution to Problem]

The present inventors have conducted studies in an attempt to achieve the above-mentioned purposes and found that a cell population predominantly containing atrial myocytes is obtained by culturing a cell population containing early cardiomyocytes in the presence of a Notch signal inhibitor, which resulted in the completion of the present invention.

To solve the above-mentioned problems, the present invention provides the following [1] to [10].
[1] A method for producing an atrial myocyte from an early cardiomyocyte, comprising (a) a step of culturing a cell population containing early cardiomyocytes in the presence of a Notch signal inhibitor.
[2] The method of [1], wherein the aforementioned early cardiomyocyte is derived from a pluripotent stem cell.
[2a] The method of [2], wherein the aforementioned pluripotent stem cell is an induced pluripotent stem cell.
[3] The method of any of [1] to [2a], wherein the aforementioned Notch signal inhibitor is a γ secretase inhibitor.
[4] The method of [3], wherein the aforementioned Notch signal inhibitor is at least one compound selected from the group consisting of LY411575, Deshydroxy LY-411575, DAPT, Dibenzazepine, L-685,458, RO4929097, compound E, Tarenflurbil, Avagacestat, Nirogacestat, Crenigacestat, MK0752, MRK003, BPN-15606, CB-103, RBPJ inhibitor 1, SAHM1, IMR-1A and IMR-1, as well as derivatives thereof, and salts, solvates and isomers thereof having Notch signal transduction pathway inhibition property.
[5] The method of any of [1] to [4], comprising (b) a step of enriching the atrial myocyte from the aforementioned cell population by using an expression level of CD151 as an index.
[6] A cell population comprising atrial myocytes, which is obtained by the method of any of [1] to [5].
[7] A method for producing an atrial myocyte from a stem cell, comprising
   (A) a step of inducing a cell population containing early cardiomyocytes from the aforementioned stem cells,
   (B) a step of culturing the aforementioned cell population containing early cardiomyocytes in the presence of a Notch signal inhibitor, and
   (C) a step of enriching the atrial myocyte from the aforementioned cell population by using an expression level of CD151 as an index.
[8] A reagent for producing an atrial myocyte from an early cardiomyocyte, comprising a Notch signal inhibitor.
[9] The reagent of [8], comprising a probe for detecting CD151.
[10] Use of a Notch signal inhibitor in the production of an atrial myocyte from an early cardiomyocyte.

### [Advantageous Effects of Invention]

According to the present invention, atrial myocytes can be produced with high efficiency from early cardiomyocytes by including a step of culturing a cell population containing early cardiomyocytes in the presence of a Notch signal inhibitor. This makes it possible to obtain a cell population predominantly containing atrial myocytes.

### [Brief Description of Drawings]

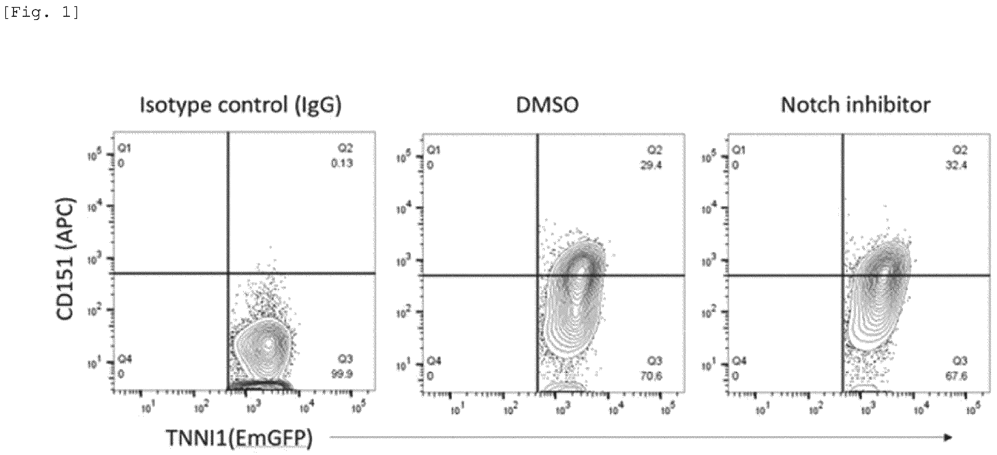

[Fig. 1] A two-dimensional flow cytometer plot of the analysis results of the expression of CD151 in EmGFP-positive cells, in which the EmGFP-positive cells were obtained by staining a cell population containing atrial myocytes, obtained by culturing a human iPS cell line with EmGFP knocked in at the TNNI1 locus in cardiomyocyte induction medium 3 containing Notch signal inhibitor (LY411575) or DMSO from day 8 to day 20 of differentiation, with an anti-CD151 antibody and a corresponding Alexa (registered trademark) 647-labeled secondary antibody. The right is a cell population cultured in cardiomyocyte induction medium 3 containing a Notch signal inhibitor (LY411575), and the center is a cell population cultured in cardiomyocyte induction medium 3 containing DMSO as a control. The left is, as a negative control, a two-dimensional flow cytometer plot of the analysis results of the expression of CD151 in EmGFP-positive cells, in which the EmGFP-positive cells were obtained by staining a cell population cultured in cardiomyocyte induction medium 3 containing DMSO, with an IgG1 isotype antibody instead of an anti-CD151 antibody and an Alexa (registered trademark) 647-labeled secondary antibody against said antibody.

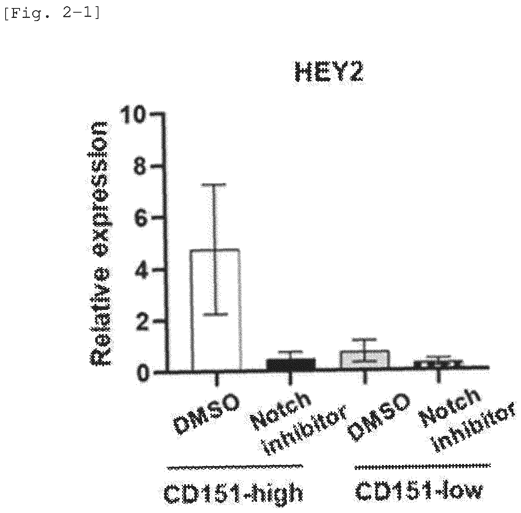

[Fig. 2-1] A bar graph showing the results of expression analysis of the target gene (HEY2) of Notch signal transduction, using total RNA extracted from each of the cell subpopulations of "DMSO/CD151-high", "DMSO/CD151-low", "Notch inhibitor/CD151-high" and "Notch inhibitor/CD151-low", obtained in Experimental Example 2. Relative values (mean±standard error) are shown in which the expression level of HEY2 in Notch inhibitor/CD151-high is 1.

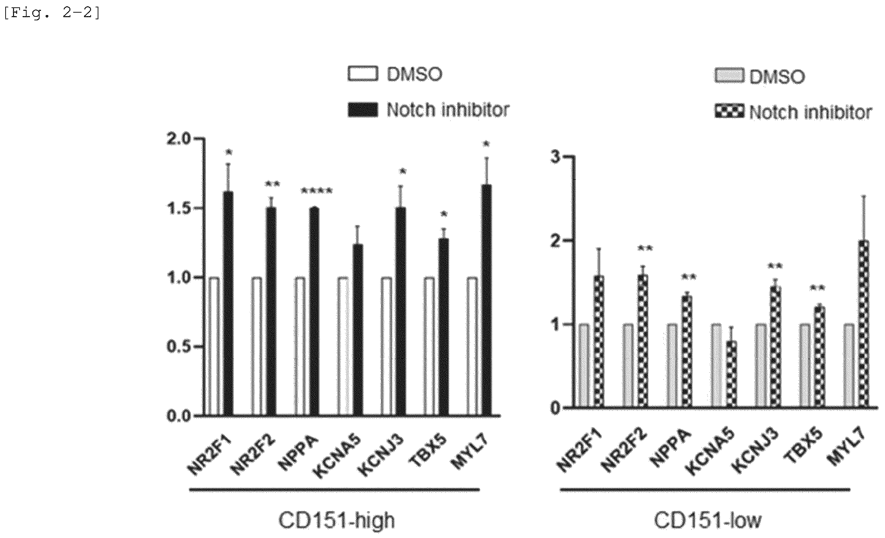

[Fig. 2-2] Bar graphs showing the results of expression analysis of the marker genes (NR2F1, NR2F2, NPPA, KCNA5, KCNJ3, TBX5 and MYL7) of atrial myocytes, using total RNA extracted from each of the cell subpopulations of "DMSO/CD151-high", "DMSO/CD151-low", "Notch inhibitor/CD151-high" and "Notch inhibitor/CD151-low", obtained in Experimental Example 2. Relative values (mean±standard error) are shown in which the expression level of each gene in DMSO-added cell is 1.

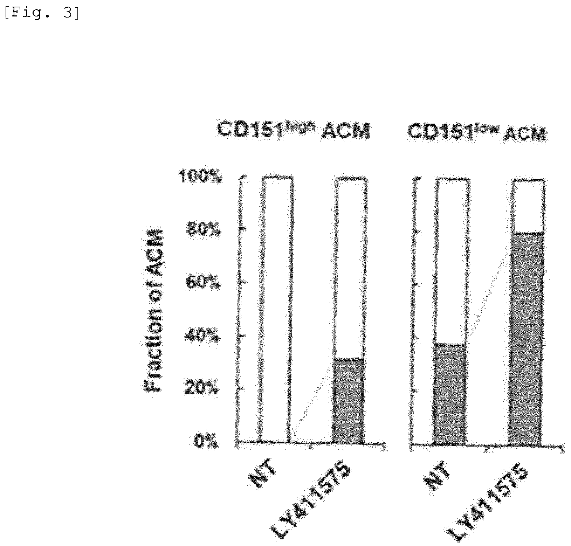

[Fig. 3] A bar graph of the proportion of atrial myocytes (ACM) and other cells in the "Notch inhibitor/CD151-high" and "Notch inhibitor/CD151-low" cardiomyocytes subpopulations obtained in Experimental Example 2, and a bar graph of the proportion of atrial myocytes (ACM) and other cells in the "No treat/CD151-high" and "No treat/CD151-low" cardiomyocytes subpopulations obtained under conditions not containing a Notch inhibitor. The graph of "CD151^{high}ACM" shows the results of "Notch inhibitor/CD151-high", in which "NT" means the condition without a Notch inhibitor, and "LY411575" means the condition with LY411575, a Notch inhibitor. The graph of "CD151^{low} ACM" shows the results of "Notch inhibitor/CD151-low", in which "NT" means the condition without a Notch inhibitor, and "LY411575" means the condition with LY411575, a Notch inhibitor. In each graph, the proportion of atrial myocytes (ACM) is indicated in grey.

### (Detailed Description of the Invention)

The present invention provides a method for producing atrial myocytes from early cardiomyocytes.

In the present specification, the "stem cell" refers to a cell that has both differentiation potency and proliferation potency (particularly self-replication potency) while maintaining differentiation potency. Stem cells include subpopulations such as pluripotent stem cells, multipotent stem cells, and unipotent stem cells depending on the differentiation ability. Pluripotent stem cells refer to stem cells that have the ability (pluripotency) to differentiate into all cells that constitute a living body (tissues derived from the three germ layers (ectoderm, mesoderm, and endoderm)). Multipotent stem cells refer to stem cells that have the ability to differentiate into multiple, though not all, types of tissues and cells. Unipotent stem cells refer to stem cells that have the ability to differentiate into specific tissues and cells. The "pluripotent stem cells" that can be used in the present invention are not particularly limited, and include, for example, embryonic stem cells (ESCs), embryonic stem cells derived from cloned embryos obtained by nuclear transfer, spermatogonial stem cells, embryonic germ cells, and induced pluripotent stem cells (sometimes referred to as "iPSCs" in the present specification). In addition, the "pluripotent stem cells" that can be used in the present invention refer to stem cells that have the ability to differentiate into a limited number of lineages of cells. Preferred pluripotent stem cells are ESC and iPSC.

As for "embryonic stem cells (ESC)", various mouse ESC strains established by inGenious targeting laboratory, RIKEN and the like can be used for mouse ESC, and various human ESC strains established by University of Wisconsin, NIH, RIKEN, Kyoto University, National Center for Child Health and Development, Cellartis and the like can be used for human ESC. For example, as human ESC strains, CHB-1 to CHB-12, RUES1, RUES2, and HUES1 to HUES28 strains distributed by ESI Bio, H1 and H9 strains distributed by WiCell Research, and KhES-1, KhES-2, KhES-3, KhES-4, KhES-5, SSES1, SSES2, and SSES3 strains distributed by RIKEN can be utilized.

The "induced pluripotent stem cell" refers to cells obtained by reprogramming mammalian somatic cells or undifferentiated stem cells by introducing specific factors (nuclear reprogramming factors). Currently, there are various types of "induced pluripotent stem cell", and iPSC established by Yamanaka et al. by introducing four factors of Oct3/4, Sox2, Klf4, and c-Myc, into mouse fibroblasts (Takahashi K, Yamanaka S., Cell, (2006) 126: 663-676), as well as human cell-derived iPSCs established by introducing the same four factors into human fibroblasts (Takahashi K, Yamanaka S., et al. Cell, (2007) 131: 861-872.), Nanog-iPSCs established by selecting using the expression of Nanog as an indicator after the introduction of the above four factors (Okita, K., Ichisaka, T., and Yamanaka, S. (2007). Nature 448, 313-317.), iPSCs produced by a method that does not include c-Myc (Nakagawa M, Yamanaka S., et al. Nature Biotechnology, (2008) 26, 101-106), iPSCs established by introducing six factors using a virus-free method (Okita K et al. Nat. Methods 2011 May; 8(5):409-12, Okita K et al. Stem Cells. 31(3):458-66.), and the like can also be used. In addition, induced pluripotent stem cells established by introducing the four factors OCT3/4, SOX2, NANOG, and LIN28 created by Thomson et al. (Yu J., Thomson JA. et al., Science (2007) 318: 1917-1920.), induced pluripotent stem cells produced by Daley et al. (Park IH, Daley GQ. et al., Nature (2007) 451: 141-146), and induced pluripotent stem cells produced by Sakurada et al. (JP-A-2008-307007) can also be used.

In addition, any of the artificial pluripotent stem cells known in the art and described in all published papers (e.g., Shi Y., Ding S., et al., Cell Stem Cell, (2008) Vol3, Issue 5,568-574; , Kim JB., Scholer HR., et al., Nature, (2008) 454, 646-650; Huangfu D., Melton, DA., et al., Nature Biotechnology, (2008) 26, No 7, 795-797), or patents (e.g., JP-A-2008-307007, JP-A-2008-283972, US2008-2336610, US2009-047263, WO2007-069666, WO2008-118220, WO2008-124133, WO2008-151058, WO2009-006930, WO2009-006997, WO2009-007852) can be used. As the artificial pluripotent stem cell line, various iPSC lines established by NIH, RIKEN, Kyoto University, and the like can be used. For example, human iPSC lines include RIKEN's HiPS-RIKEN-1A line, HiPS-RIKEN-2A line, HiPS-RIKEN-12A line, Nips-B2 line, and the like, and Kyoto University's 253G1 line, 201B7 line, 409B2 line, 454E2 line, 606A1 line, 610B1 line, 648A1 line, 1231A3 line, 1390D4 line, and 1390C1 line, and the like, and 1390D4 line and 1390C1 line are more preferred. Alternatively, clinical grade cell lines provided by Kyoto University, Cellular Dynamics International, and the like, and research and clinical cell lines prepared using these cell lines, and the like may also be used.

The "cardiomyocyte" refers to the smallest unit of cells that constitute myocardium and have contractile ability, and includes atrial cardiomyocyte, ventricular cardiomyocyte, and the like.

Identification of cells as atrial myocytes or ventricular myocytes can be performed by conventionally known techniques, such as electrophysiological analysis, marker expression analysis, and drug response analysis, described below.

For example, any of the following (1) to (7) using the patch clamp method can be used for identification by electrophysiological analysis.

(1) Cells that show an action potential waveform with a ratio (APD30/90) of the action potential duration at 30% repolarization (APD30) to the action potential duration at 90% repolarization (APD90) of 0.3 or more and a maximum rate of rise of the waveform (dv/dtmax) of 10 or more are defined as ventricular myocytes. On the other hand, cells that show an action potential waveform with an APD30/90 of less than 0.3 and a dv/dtmax of 10 or more are defined as atrial myocytes. In this case, cells that show an action potential waveform with a dv/dtmax of less than 10 and a spontaneous action potential period of 1s or more are defined as immature cardiomyocytes that are not classified as either ventricular or atrial myocytes (Cell Stem Cell, 2017, 21, 179-194).
(2) Cells that show an action potential waveform with a significantly long value of the action potential duration at 50% repolarization (APD50) or APD90 are defined as ventricular myocytes, and cells that show a significantly short action potential waveform are defined as atrial myocytes (JCI Insight, 2018; 3(12):e99941, Eur. Heart J. 2017, 38, 292-301).
(3) Cells that show an action potential waveform with a significantly long value of the action potential duration at 20% repolarization (APD20), APD50, or APD90 and a significantly larger amplitude of the action potential plateau phase (APAPlat) are defined as ventricular myocytes, and cells that show an action potential waveform with a significantly short APD20, APD50, or APD90 value and significantly small APAPlat are defined as atrial myocytes (Stem Cell Reports. 2017 Dec 12; 9(6): 1765-1779).
(4) Cells that show an action potential waveform with a significantly large ratio of APD20 to the action potential duration at 80% repolarization (APD80) (APD20/80) are defined as ventricular myocytes, and cells that show an action potential waveform with a significantly small ratio are defined as atrial myocytes (JCI Insight, 2018; 3(12):e99941).
(5) Cells that show an action potential waveform with a significantly small ratio of APD90 to APD50 (APD90/50) are defined as ventricular myocytes, and cells that show an action potential waveform with a significantly large ratio are defined as atrial myocytes (Eur. Heart J., 2017, 38, 292-301, Eur. Heart J., 2011, 32, 952-962).
(6) Cells that show an action potential waveform with an APD90/50 of less than 1.4 are defined as ventricular myocytes, and cells that show an action potential waveform with an APD90/50 of more than 1.7 are defined as atrial myocytes. In this case, cells that show an action potential waveform with an APD90/50 of 1.4 or more and 1.7 or less are defined as pacemaker cells (Eur. Heart J., 2011, 32, 952-962).
(7) Cells that show an action potential waveform with a plateau phase of 50 ms or more with a membrane potential change of 20 mV or less, dv/dtmax greater than 50, action potential amplitude (APA) greater than 85 mV, and APD90/50 less than 2.3 are defined as ventricular myocytes. Cells that show an action potential waveform that differs from the above action potential waveform only in that it lacks a plateau phase are defined as atrial myocytes, and cells that show an action potential waveform that differs in that it lacks a plateau phase and has an APD90/50 greater than 2.3 are defined as pacemaker cells (PNAS, 2017, E8372-E8381).

Identification by marker expression analysis involves measuring the expression of marker proteins or marker genes, and when a cell strongly expresses an atrial myocyte marker and preferably does not express or weakly expresses a ventricular myocyte marker, the cell can be determined to be an atrial myocyte. When the expression of a marker gene can be quantitatively measured, the relative expression value corrected by the expression amount of a housekeeping gene can be calculated, and a cell with a high relative expression amount of an atrial myocyte marker and a low relative expression amount of a ventricular myocyte marker can be determined to be an atrial myocyte. Conversely, when a cell expresses a ventricular myocyte marker and preferably does not express an atrial myocyte marker, the cell can be determined to be a ventricular myocyte. Alternatively, when the expression of a marker gene can be quantitatively measured, the relative expression value corrected by the expression amount of a housekeeping gene can be calculated, and a cell with a high relative expression amount of a ventricular myocyte marker and a low relative expression amount of atrial myocyte marker can be determined to be a ventricular myocyte. Known marker genes for atrial myocytes include KCNA5 (Potassium voltage-gated channel subfamily A member 5), KCNJ3 (Potassium voltage-gated channel subfamily J member 3), NPPA (Natriuretic peptide A), NR2F1 (Nuclear receptor subfamily 2 group F member 1), NR2F2 (Nuclear receptor subfamily 2 group F member 2), TBX5 (T-Box 5), and MYL7 (Myosin Light Chain 7), and known marker genes for ventricular myocyte include HEY2 (Hes related family BHLH transcription factor with YRPW motif 2), MYL2 (Myosin light chain2), and IRX4 (Iroquois homeobox 4). As housekeeping genes, GAPDH, β-actin, and the like are known. In order to ensure more accuracy, a housekeeping gene that does not fluctuate much during differentiation of early cardiomyocytes into atrial myocytes and/or ventricular myocytes is selected, and the expression levels of the marker genes of ventricular myocytes and atrial myocytes are corrected with the expression levels of the housekeeping gene. For example, software such as geNorm, described in Joey St-Pierre et al. (Scientific Reports 7: 16923 (2017)), can be used to select the housekeeping gene for correction.

In addition, cardiomyocytes including atrial myocytes and ventricular myocytes refer to cells expressing at least one cardiomyocyte marker (at least one marker selected from the group consisting of cardiac troponin (cTNT), αMHC (α myosin heavy chain, MYH6), βMHC (MYH7) and NKX2.5 (NK-2 transcription factor related, locus 5)). The cTNT gene is exemplified by NCBI accession number NM_000364 in human and NM_001130176 in mouse. The αMHC gene is exemplified by NCBI accession number NM_002471 in human and NM_001164171 in mouse. The βMHC gene is exemplified by NCBI accession number NM_000257 in human and NM_080728 in mouse.

Marker proteins can be detected by immunological assays using antibodies specific to the marker proteins, such as ELISA, immunostaining, flow cytometry, and the like. Marker genes can be detected by nucleic acid amplification methods and/or nucleic acid detection methods known in the art, such as RT-PCR, microarray, biochip, and the like.

Identification by drug response analysis includes detecting the response of cells to drugs that activate or inhibit channels that are specifically expressed in atrial myocyte or ventricular myocyte. For example, carbachol is a drug that activates muscarinic K channels (IK, Ach) that are specifically expressed in atrial myocytes. When carbachol is added to cells, it shortens the action potential duration (APD) only in atrial myocytes, but does not affect the APD of ventricular myocytes. In addition, 4-aminopyridine is a drug that inhibits ultrarapidly activated delayed rectifier K channels (IKur). When it is added to cells, it prolongs APD20 only in atrial myocytes, but does not affect the APD of ventricular myocytes (Stem Cell Reports, 2018; 11(6):1378-1390.). Atrial myocyte or ventricular myocyte can also be identified by detecting the responsiveness of cells to such drugs.

The method of the present invention for producing atrial myocytes from early cardiomyocytes includes (a) a step of culturing a cell population containing early cardiomyocytes in the presence of a Notch signal inhibitor.

In the present specification, the "early cardiomyocyte" is a cell at a stage where it is oriented to a cardiomyocyte in the process of differentiating from a non-cardiomyocyte to a cardiomyocyte, and refers to, for example, a cell at a stage where any one or more of myocardial-specific genes (cTNT, NKX2.5, MYH6, MYH7, MYL7, etc.), the above-described atrial muscle marker genes (other than KCNJ3), and the above-described ventricular muscle marker genes (other than MYL2) begin to be expressed. Early cardiomyocytes may be derived from stem cells, for example, embryonic stem cells, adult stem cells, and iPS cells, or may be early cardiomyocytes directly isolated from a living body. Early cardiomyocytes are preferably derived from pluripotent stem cells, more preferably from iPS cells. For example, when atrial myocytes are produced from iPS cells under atrial myocyte differentiation conditions known in the art (see, for example, EMBO Mol Med (2015) 7:394-410 and Patent Literature 1), embryoid bodies produced from iPSCs are cultured for two days in a basal medium containing BMP4, activin A, and bFGF, and then cultured for three days in a basal medium containing VEGF, a Wnt inhibitor, a TGF-β inhibitor, and retinoic acid. Furthermore, by culturing in a basal medium containing VEGF, the differentiation of atrial myocytes is oriented. In this method, specifically, the expression of Notch 4 increases in early cardiomyocytes on about day 8, with the start of iPS cell culture being day 0. In the method of producing atrial myocytes from early cardiomyocytes of the present invention, a cell population containing early cardiomyocytes is cultured in the presence of a Notch signal inhibitor.

In the present specification, the "cell population" means a collection of two or more cells. The "cell subpopulation" means a collection of cells that constitute a cell population and that have at least one common characteristic.

The "culture" refers to maintaining, propagating (growing) and/or differentiating cells in an in vitro environment. "Culturing" refers to maintaining, propagating (growing) and/or differentiating cells outside a tissue or outside the body in, for example, a cell culture dish, plate, flask or culture tank.

The "maintenance culture (Sustain)" refers to culturing a desired cell population while maintaining the number thereof. The maintenance of cell number may be achieved by cells surviving without proliferation, or by balancing the increase in number due to proliferation and the decrease in number due to death of cells. The maintenance of cell number does not need to be achieved by maintaining the exact same number of cells, but it is sufficient that the number of cells is substantially the same for the purposes of the present invention.

For culturing the cell population, the basal medium is not particularly limited, but, for example, StemPro-34 SFM (Thermo Fisher Scientific), STEMdiff APEL2 medium (STEMCELL Technologies, ST-05275), TeSR1 medium and Chemically Defined medium (CDM) are preferably used. In addition, BME medium, BGJb medium, CMRL 1066 medium, Glasgow MEM medium, Improved MEM (IMEM) medium, Improved MDM (IMDM) medium, Medium 199 medium, Eagle MEM medium, αMEM medium, DMEM medium (High glucose, Low glucose), DMEM/F12 medium, Ham's medium, RPMI 1640 medium, Fischer's medium, and mixed media thereof may also be used. The CDM medium is not particularly limited, but, for example, a medium prepared from Iscove's modified Dulbecco's medium (GE Healthcare) may be used. The basal medium may be supplemented with substances generally used in cell culture, such as Ham's F-12 nutrient mixture, albumin such as human serum albumin, polyvinyl alcohol (PVA), deionized BSA, linoleic acid, linolenic acid, cholesterol, insulin, apotransferrin, selenium, ethanolamine, monothioglycerol, protein-free hybridoma mixture II (PFHMII), ascorbic acid, L-alanyl-L-glutamine, and/or antibiotics.

In the present specification, the Notch signal inhibitor refers to a drug that inhibits the signal transduction pathway activated by direct interaction between the Notch protein, which is a receptor expressed on the cell membrane, and a Notch ligand (such as Delta or Jagged) expressed on the membrane of an adjacent cell.

The Notch signal inhibitor is not particularly limited as long as it can suppress the signal transduction mediated by Notch. It may be any of nucleic acids, proteins, and low molecular weight organic compounds. Examples of the substance include function-defective Notch receptors and ligands, substances that inhibit Notch processing (S1 cleavage), substances that inhibit the glycosylation of Notch and Notch ligands, substances that inhibit cell membrane migration, substances that inhibit the processing (S2 cleavage, S3 cleavage) of the intracellular domain (NICD) of Notch (γ-secretase inhibitors), substances that degrade NICD, and substances that inhibit NICD-dependent transcription.

Among the Notch signal inhibitors, many γ secretase inhibitors are known. This is because γ secretase, which is involved in the processing (S2 cleavage, S3 cleavage) of the intracellular domain (NICD) of Notch, is also involved in the production of amyloid beta protein, which is involved in Alzheimer's disease. Examples of gamma secretase inhibitors that can be used in the present invention include LY411575, Deshydroxy LY-411575, DAPT, Dibenzazepine, L-685,458, RO4929097, compound E, Tarenflurbil, Avagacestat, Nirogacestat, Crenigacestat, MK0752, CB-103, MRK003, BPN-15606 and the like, but are not limited to these as long as the activity of Notch signal inhibitor is present.

Of these, LY411575 (CAS No. number 209984-57-6) is the most potent γ secretase inhibitor and one of the most potent Notch signal inhibitors. Deshydroxy LY-411575 (CAS No. 209984-56-5) is a derivative of LY411575 and is also known by other names such as γ-secretase inhibitor (GSI)-XX and the like. DAPT (CAS No. 208255-80-5) is an abbreviation of N-[2S-(3,5-difluorophenyl)acetyl]-L-alanyl-2-phenyl-1,1-dimethylethyl ester-glycine, and is also known by other names such as γ-secretase inhibitor (GSI)-IX, LY-374973 and the like. Dibenzazepine (CAS No. 209984-56-5) is also known by other names such as YO-01027 and the like. The chemical name of L-685,458 (CAS No. 292632-98-5) is (5S)-(t-Butoxycarbonylamino)-6-phenyl-(4R)hydroxy-(2R)benzylhexanoyl)-L-leu-L-phe-amide. RO4929097 (CAS No. 847925-91-1) is also known by other names such as KK8645V7LE, RG-4733 and the like. Compound E (CAS No. 209986-17-4) is also known by other names such as γ- secretase inhibitor (GSI)-XXI and the like. Tarenflurbil (CAS No. 51543-40-9) is also known by other names such as (R)-Flurbiprofen and the like. Avagacestat (CAS No. 1146699-66-2) is also known by other names such as BMS-708163 and the like. Nirogacestat (CAS No. 1290543-63-3) is also known by other names such as PF-3084014 hydrobromide and the like. Crenigacestat (CAS No. 1421438-81-4) is also known by other names such as LY3039478 and the like. MK0752 (CAS No. 471905-41-6) is also known by other names such as UNII-9JD9B4S53T and the like. The chemical name of MRK003 (CAS No. 623165-93-5) is (4R,10'S)-2-(2,2,2-trifluoroethyl)-5'-[(E)-3-[4-(trifluoromethyl)piperidin-1-yl]prop-1-enyl]spiro[1,2,5-thiadiazolidine-4,13'-tricyclo[8.2.1.03,8]trideca-3(8),4,6-triene] 1,1-dioxide. BPN-15606 (CAS No. 1914989-49-3) and is also known by other names such as HY-117482 and the like.

Among the Notch signal inhibitors, CB-103 (CAS No. 218457-67-1) is not a γ-secretase inhibitor, but a substance that inhibits NICD-dependent transcription by preventing Notch intracellular domain (NICD) from migrating into the nucleus to form a transcription activation complex with γ-secretase.

RBPJ inhibitor 1 (CAS No. 2682114-39-0) suppresses Notch-mediated signal transduction by blocking the functional interaction of RBPJ, which is a major effector of Notch signal transduction, with the scaffolding protein SHARP.

SAHM1 (CAS No. 2050906-89-1), IMR-1A (CAS No. 331862-41-0) and IMR-1 (CAS No. 310456-65-6) are all dominant-negative mutant peptidomimetics of the mastermind-like (MAML) protein, and suppress Notch-mediated signal transduction.

In the present invention, the concentration of the Notch signal inhibitor added to the culture medium for the cell population containing early cardiomyocytes is generally 0.1 µM or more, preferably 1 µM or more. There is no particular upper limit to the concentration of the Notch signal inhibitor as long as it does not adversely affect differentiation into atrial myocytes. From the aspect of culture costs, it is generally 1000 µM or less, preferably 100 µM or less. In one embodiment, the concentration of the Notch signal inhibitor in the medium is generally 0.1 to 1000 µM, preferably 1 to 100 µM (e.g., 10 µM).

In the present invention, the time to start adding a Notch signal inhibitor to the culture medium of a cell population containing early cardiomyocytes is when the expression of Notch 4 increases in the early cardiomyocytes, for example, around day 8, with the start of iPS cell culture being day 0, under conventionally known atrial myocyte differentiation conditions (see, for example, EMBO Mol Med (2015) 7:394-410), but a different time may be appropriate depending on the conditions.

The period of adding a Notch signal inhibitor to the culture medium of a cell population containing early cardiomyocytes is not particularly limited. It is, for example, 7 to 40 days, and may be, for example, 120 days, 90 days, 60 days, 30 days, 28 days, 21 days, 14 days, or 7 days depending on the purpose.

In the present invention, when a cell population containing early cardiomyocytes is induced from stem cells, the method is described, for example, in Patent Literature 1. Here, the atrial myocyte differentiation conditions include, for example, culturing an embryoid body created from iPSCs in a basal medium containing BMP4, activin A, and bFGF for two days, and then culturing in a basal medium containing VEGF, a Wnt inhibitor, a TGF-β inhibitor, and retinoic acid for three days. A cell population containing atrial myocytes can be obtained by further culturing in a basal medium containing VEGF.

Wnt inhibitors (Wnt signal inhibitors) are substances that inhibit the signal transduction pathway mediated by Wnt and, for example, IWP-2, IWP-3, IWP-4, 2-(4-trifluoromethylphenyl)-7,8-dihydro-5H-thiopyrano[4,3-d]pyrimidin-4(3H)-one, IWR1, G-CSF, IGFBP4, Dkk1, Cerberus, anti-Wnt antibodies, Wnt agonists (Wnt receptor inhibitors), soluble Wnt receptor proteins (Frzb-1, etc.), dominant negative forms, and the like can be used. Two or more of these may be used in combination.

Examples of the TGF-β inhibitor include SB431542 (4-[4-(1,3-benzodioxol-5-yl)-5-(2-pyridinyl)-1H-imidazol-2-yl]-benzamide), A83-01 (4-[4-(1,3-benzodioxol-5-yl)-5-(2-pyridinyl)-1H-imidazol-2-yl]-benzamide), LDN193189 (4-[6-[4-(1-Piperazinyl)phenyl]pyrazolo[1,5-a]pyrimidin-3-yl]-quinoline), GW788388 (4-[4-[3-(2-Pyridinyl)-1H-pyrazol-4-yl]-2-pyridinyl]-N-(tetrahydro-2H-pyran-4-yl)-benzamide), SM16 (4-[4-(1,3-Benzodioxol-5-yl)-5-(6-methyl-2-pyridinyl)-1H-imidazol-2-yl]-bicyclo[2.2.2]octane-1-carboxamide), IN-1130 (3-[[5-(6-Methyl-2-pyridinyl)-4-(6-quinoxalinyl)-1H-imidazol-2-yl]methyl]-benzamide), GW6604 (2-Phenyl-4-[3-(pyridin-2-yl)-1H-pyrazol-4-yl]pyridine), SB505124 (2-[4-(1,3-Benzodioxol-5-yl)-2-(1,1-dimethylethyl)-1H-imidazol-5-yl]-6-methyl-pyridine) and the like. Two or more of these may be used in combination.

The method of producing atrial myocytes from early cardiomyocytes of the present invention may include, in addition to or subsequent to the aforementioned steps (a), (b), a step of enriching atrial myocytes from the aforementioned cell population by using the expression level of CD151 as an index.

"Enrich" and "enrichment" refer to increasing the amount of a specific constituent component in a composition, such as a cell composition. "Enriched", when used to describe a composition of cells, e.g., a cell population, refers to an increase in the amount of a specific constituent component compared to the proportion of such component in the cell population prior to enrichment. For example, a composition, such as a cell population, can be enriched for a target cell type, such that the proportion of the target cell type is increased compared to the proportion of the target cells present in the cell population prior to enrichment. Cell populations can also be enriched for a target cell type by cell selection and sorting methods known in the art. Cell populations can also be enriched by certain selection or sorting processes described in the present specification. In a specific embodiment of the present invention, the cell population after enrichment is at least 1%, 2%, 3%, 4%, 5%, 6%, 7%, 8%, 9%, 10%, 20%, 30%, 40%, 50%, 60%, 70%, 80%, 85%, 90%, 95%, 97%, 98% or 99% enriched with respect to the target cell population relative to the cell population before enrichment. In a specific embodiment of the present invention, the cell population after enrichment contains at least 1%, 2%, 3%, 4%, 5%, 6%, 7%, 8%, 9%, 10%, 20%, 30%, 40%, 50%, 60%, 70%, 80%, 85%, 90%, 95%, 97%, 98%, 99% or 100% of the target cell population. In the present specification, the "cell population predominantly containing atrial myocytes" may refer to a cell population that contains at least 80%, 85%, 90%, 95%, 97%, 98%, 99% or 100% atrial myocytes.

In a cell population derived from stem cells and including cardiomyocytes, the cell subpopulation identified by high CD151 expression contains a higher proportion of ventricular myocytes than the cell subpopulation identified by low CD151 expression. Thus, atrial myocytes can be enriched in a cell population by removing the cell subpopulation identified by high CD151 expression from the cell population derived from stem cells and including cardiomyocytes, or by recovering the cell subpopulation identified by low CD151 expression from the cell population including cardiomyocytes.

"CD151" is a four-transmembrane protein belonging to the tetraspanin family. CD151 plays an important role in signal transduction related to cell differentiation, proliferation, and movement. CD151 is also known to form a complex with integrin and be involved in functions such as cell adhesion and fusion. In the present invention, CD151 is used as a marker for enriching atrial myocyte or ventricular myocyte in a cell population derived from stem cells and including cardiomyocytes.

"Marker" is a "marker protein" or a "marker gene" and refers to a protein that is specifically expressed on the cell surface, in the cytoplasm, and/or in the nucleus of a given cell type, or a gene thereof. The marker may be a positive selection marker or a negative selection marker. Preferably, the marker is a cell surface marker, and a cell surface selection marker enables concentration, isolation, and/or detection of viable cells.

Marker proteins can be detected by immunological assays using an antibody specific to the marker protein, such as ELISA, immunostaining, and flow cytometry. As an antibody specific to a marker protein, an antibody that binds to a specific amino acid sequence in the marker protein or a specific sugar chain bound to the marker protein can be used. In addition, in the case of a marker protein that is expressed intracellularly and is not present on the cell surface (e.g., a transcription factor or a subunit thereof), a reporter protein is expressed together with the marker protein, and the reporter protein is detected to detect the marker protein of interest. This method can be preferably used when no suitable cell surface marker is found. Marker genes can be detected using a nucleic acid amplification method and/or a nucleic acid detection method known in the art, such as RT-PCR, microarray, biochip, and RNA-seq.

"Expression" is defined as the transcription of a specific nucleotide sequence and/or translation of the transcript driven by a promoter.

"Positive" or "expressed" means that the protein or mRNA is expressed in an amount detectable by techniques known in the art. Proteins can be detected by immunological assays using antibodies, such as ELISA, immunostaining, and flow cytometry. In addition, in the case of a protein that is expressed intracellularly and is not present on the cell surface (e.g., a transcription factor or a subunit thereof), a reporter protein is expressed together with the protein, and the reporter protein is detected to detect the protein of interest. mRNA can be detected using a nucleic acid amplification method and/or a nucleic acid detection method, such as RT-PCR, microarray, biochip, and RNA-seq.

"Negative" or "not expressed" means that the expression level of a protein or gene is below the lower limit of detection by all or any of the known techniques described above. The lower limit of detection of protein or gene expression may vary for each technique.

The expression level of CD151 in cells can be determined by setting a certain reference value based on the distribution of expression level in a cell population, and determining that cells exhibiting an expression level equal to or greater than the reference value are highly expressed, and that cells exhibiting an expression level less than the reference value are low expressed. In this case, the reference value may be, for example, the maximum value, average value, median value or mode value of the expression level of each cell, preferably the maximum value. The reference value can be set appropriately depending on the desired cell enrichment rate, and can be set to, for example, a value that is 1%, 2%, 3%, 4%, 5%, 10%, 15%, 20%, 30%, 40%, 50%, 60%, 70%, 80%, 90%, 100% or more greater or smaller than the maximum, average, median, or mode (preferably the maximum) of the expression level of each cell.

The expression level of CD151 in cells can also be determined by setting the expression level in stem cells as a reference value, and determining that cells exhibiting an expression level equal to or greater than the reference value are highly expressed, and that cells exhibiting an expression level less than the reference value are low expressed. In this case, the reference value may be, for example, the maximum value, average value, median value or mode value of the expression level in stem cells, preferably the maximum value. The reference value can be set appropriately depending on the desired cell enrichment rate, and can be set to, for example, a value that is 1%, 2%, 3%, 4%, 5%, 10%, 15%, 20%, 30%, 40%, 50%, 60%, 70%, 80%, 90%, 100% or more greater or smaller than the maximum, average, median, or mode (preferably the maximum) of the expression level in the stem cells.

Alternatively, the expression level of CD151 in cells can also be determined by setting the signal intensity detected in cells not contacted with the probe (negative control) in the same manner as in the cells contacted with the probe as a reference value, and determining that cells exhibiting a signal intensity equal to or greater than the reference value are highly expressed, and that cells exhibiting a signal intensity less than the reference value are low expressed. The reference value can be set appropriately depending on the desired cell enrichment rate, and can be set to, for example, a value that is 1%, 2%, 3%, 4%, 5%, 10%, 15%, 20%, 30%, 40%, 50%, 60%, 70%, 80%, 90%, 100% or more greater or smaller than the maximum, average, median, or mode (preferably the maximum) of the expression level of the negative control.

The recovery of the target cell subpopulation or the removal of the non-target cell subpopulation can be performed under conventionally known conditions and, for example, cell sorting using a flow cytometer can be suitably applied. As an example, first, the cells are reacted with an anti-CD151 primary antibody. The cells are washed to remove the anti-CD151 primary antibody that is not bound to the cells, and then the cells are reacted with a fluorescently-labeled secondary antibody. After further washing the cells to remove the fluorescently labeled secondary antibody that is not bound to the primary antibody, the fluorescence intensity of the cells is measured using a flow cytometer, and cells that show an expression level equal to or higher than the above-mentioned reference value are sorted into a CD151 high expression cell subpopulation (CD151-high), and cells that show an expression level below the reference value are sorted into a CD151 low expression cell subpopulation (CD151-low).

The present invention provides a cell population containing atrial myocytes, obtained by the method of the present invention. As used herein, the "atrial myocyte" and "cell population" are as described above.

The present invention provides a method for producing an atrial myocyte from a stem cell, including
(A) a step of inducing a cell population containing early cardiomyocytes from the aforementioned stem cells,
(B) a step of culturing the aforementioned cell population containing early cardiomyocytes in the presence of a Notch signal inhibitor, and
(C) a step of enriching the atrial myocyte from the aforementioned cell population by using an expression level of CD151 as an index. Each step of the method of the present invention is as described above.

The present invention provides a reagent for producing atrial myocytes from stem cells. The reagent of the present invention includes a Notch signal inhibitor. The "Notch signal inhibitor" is as described above.

The reagent of the present invention may include a probe for detecting CD151.

The expression level of CD151 in cells can be determined using a CD151 detection probe having binding property to CD151 (hereinafter to be also simply referred to as "probe"). The probe may be, for example, an antibody or a nucleic acid (such as an aptamer), and is preferably an antibody. The probe is preferably labeled such that it is optically, electrically, or magnetically detectable. In this case, the cell is contacted with the probe, and the signal from the label of the probe bound to CD151 is optically, electrically, or magnetically detected, and the expression level of CD151 in cells can be determined based on the signal intensity. In addition, for example, when the probe is an antibody, the expression level of CD151 in cells can also be determined using a labeled secondary antibody that binds to the antibody.

In addition, for example, when the probe is an antibody, the expression level of CD151 in cells can also be determined using a labeled secondary antibody that binds to the antibody.

The present invention provides use of a Notch signal inhibitor for the production of atrial myocytes from early cardiomyocytes. The Notch signal inhibitor here may be provided in a state where it is added to a culture solution for a cell population containing early cardiomyocytes. From the viewpoint of storage stability, it may be provided in a dry form or in the form of a highly concentrated stock solution and may be prepared when in use.

The atrial myocytes and cell populations containing atrial myocytes produced by the present invention can be applied to cell medicines containing the atrial myocytes for treating cardiac disease, and methods for treating cardiac diseases by administering the cell medicine. The present invention also provides use of the aforementioned cell population and atrial myocytes for producing the aforementioned cell medicine, and the aforementioned cell population and atrial myocytes for use in treating cardiac diseases.

The cell medicine containing atrial myocytes and a cell population containing atrial myocytes produced by the present invention can be used in regenerative medicine for atrial muscle-specific diseases such as atrial fibrillation, and cardiac diseases such as myocardial infarction, heart failure, ischemic heart disease, cardiomyopathy, myocarditis, hypertrophic cardiomyopathy, dilated phase hypertrophic cardiomyopathy, and dilated cardiomyopathy.

The cell medicine containing atrial myocytes and a cell population containing atrial myocytes produced by the present invention is produced as a parenteral preparation in any form, such as a suspension in which cells are suspended in a suitable solvent, a cell aggregate, and a cell sheet formed into a single layer or two or more layers. The solvent may be water, physiological saline, or various buffer solutions or cell preservation solutions. The cell aggregate and cell sheet may be composed of cells alone or may be composed of a suitable biocompatible material and cells. Examples of biocompatible materials include collagen, polyglycolic acid (PGA), polylactic acid, alginate, polyethylene oxide, polylactic acid-polyglycolic acid copolymer, proteoglycan, glycosaminoglycan, human dermis, or a combination thereof. The biocompatible material may be a membrane such as a sheet, a porous body such as a sponge, or a mesh such as a woven fabric, cloth, or nonwoven fabric.

The cell medicine containing atrial myocytes and a cell population containing atrial myocytes produced by the present invention may contain other components such as pharmaceutically acceptable carriers and additives according to known means (e.g., methods described in the Japanese Pharmacopoeia, etc.) depending on the use and form. Examples of carriers and additives include isotonicity agents, thickeners, sugars, sugar alcohols, antiseptics (preservatives), antimicrobial agents or antibacterial agents, pH adjusters, stabilizers, chelating agents, oily bases, gel bases, surfactants, suspending agents, fluidizing agents, dispersing agents, buffering agents, antioxidants, and the like.

A method for treating the above-mentioned diseases is provided, which includes administering a therapeutically effective amount of a cell medicine containing atrial myocytes and a cell population containing atrial myocytes produced by the present invention to a patient.

The therapeutically effective amount is the amount of cells that, when administered to a patient, can provide a therapeutic effect against the above-mentioned diseases, compared to a control that does not receive the cells. A specific therapeutically effective amount can be appropriately determined depending on the administration form (e.g., cell suspension, cell aggregate, cell sheet), administration method (e.g., injection, transplantation), purpose of use, and the age, weight, symptoms, etc. of the patient. The effective amount per treatment for human (e.g., adult) is, for example, 200,000 to 1,000,000 cells/kg body weight.

The pharmaceutical agent of the present invention is applied to mammals including human.

The present invention is described in more detail in the following with reference to Examples, but the present invention is not limited by the Examples.

In the present specification, the conjunction "about" or "approximately" used to modify numerical values indicates a value that varies by plus or minus 30%, 25%, 20%, 15%, 10%, 8%, 6%, 5%, 4%, 3%, 2%, or 1%, respectively, from a reference value. Preferably, the term "about" or "approximately" indicates a range of plus or minus 15%, 10%, 5%, or 1%, respectively, from a reference value.

All documents referred to in the present specification are incorporated herein in their entirety by reference.

The examples of the present invention described below are for illustrative purposes only and are not intended to limit the technical scope of the present invention. The technical scope of the present invention is limited only by the claims. Modifications of the present invention, such as additions, deletions, and substitutions of the constituent elements of the present invention, may be made without departing from the spirit of the present invention.

### [Example]

### [Experimental Example 1: Induction of atrial myocyte population from iPSC]

A cell population containing cardiomyocytes was induced from iPSC under atrial myocyte differentiation conditions or ventricular myocyte differentiation conditions. As iPSC, a double knock-in human iPS cell line (1390D4 strain) was used, in which EmGFP was inserted into the TNNI1 locus and mCherry was inserted into the TNNI3 locus as reporter proteins.

Maintenance culture of iPSC was performed according to a conventionally known method ("A novel efficient feeder-free culture system for the derivation of human induced pluripotent stem cells", Masato Nakagawa, et al., Scientific Reports, 2014, 4:3594).

To differentiate iPSC into cardiomyocytes, iPSC was seeded into low-adhesion 6-well plates (2 x 10⁶ cells/1.5 ml/well) and statically cultured at 37°C and 5% oxygen to form embryoid bodies (day 0). The culture medium used was StemPro-34 SFM (Thermo Fisher Scientific) supplemented with 1% L-glutamine, 150 µg/ml transferrin, 50 µg/ml ascorbic acid, 4 x 10⁻⁴ M monothioglycerol, 10 µM Rock inhibitor (Y-27632), 2 ng/ml BMP4, and 0.5% Matrigel.

The next day (day 1), 1.5 ml/well of atrial myocyte induction medium 1 was added to each well where the cells were seeded, and the cells were cultured for another 2 days at 37°C and 5% oxygen. Atrial myocyte induction medium 1 used was StemPro-34 SFM supplemented with 1% L-glutamine, 150 µg/ml transferrin, 50 µg/ml ascorbic acid, 4 x 10⁻⁴ M monothioglycerol, 4 ng/ml BMP4, 8 ng/ml activin A, and 10 ng/ml bFGF.

Then (on the third day), the medium was replaced with atrial myocyte induction medium 2, and the cells were cultured for three days at 37°C under 5% oxygen conditions. As atrial myocyte induction medium 2, StemPro-34 SFM supplemented with 1% L-glutamine, 150 µg/ml transferrin, 50 µg/ml ascorbic acid, 4 x 10⁻⁴ M monothioglycerol, 10 ng/ml VEGF, 1 µM Wnt inhibitor (IWP-3), 5.4 µM TGF-β inhibitor (SB431542), and 1 µM retinoic acid was used.

On the 6th day of differentiation, the medium was replaced with cardiomyocyte induction medium 3, and from the 8th day of differentiation onwards, 3 µM of γ-secretase inhibitor (LY411575) that inhibits Notch signaling was added when changing the medium, and the cells were cultured at 37°C under 5% oxygen conditions until the 10th day. Cells added with 0.1% DMSO were also prepared as a control. Thereafter, the medium was changed on the 10th, 13th, 15th, and 17th days, and after the medium change on the 10th day, the cells were cultured under normal oxygen conditions until the 20th day. As the cardiomyocyte induction medium 3, StemPro-34 SFM supplemented with 1% L-glutamine, 150 µg/ml transferrin, 50 µg/ml ascorbic acid, 4 x 10⁻⁴ M monothioglycerol, and 5 ng/ml VEGF was used.

### [Experimental Example 2: Sorting of atrial myocyte population based on CD151 expression level]

On the 20th day of culture, the cell population containing atrial myocytes was dispersed into single cells and the cell number was counted. Anti-CD151 antibody (BD) was added to the cell suspension and left to stand at 4°C for 30 min. A sample with IgG1 isotype antibody added was also prepared as a negative control. After washing, Alexa (registered trademark) 647-labeled secondary antibody was added to the cell suspension and the cells were left standing at 4°C for 30 min. CD151 expression in EmGFP-positive cells was analyzed using a flow cytometer (BD FACS Aria Fusion cell sorter). The fluorescence intensity of 95% or more of the negative control cells was set as the threshold, and the cell subpopulations showing fluorescence intensity lower than the threshold were defined as "CD151-low" and the cell subpopulations showing fluorescence intensity higher than the threshold were defined as "CD151-high". The cell subpopulations were then sorted into "DMSO/CD151-high", "DMSO/CD151-low", "Notch inhibitor/CD151-high", and "Notch inhibitor/CD151-low". The plot of the flow cytometer is shown in Fig. 1, and the proportion of the cell subpopulations are shown in Table 1. Values are shown as the mean±standard error of the experimental data.

**[Table 1]**

| | DMSO | | Notch inhibitor | |
|---|---|---|---|---|
| | CD151-high | CD151-low | CD151-high | CD151-low |
| Cell population (%) | 36.0±4.0 | 64.0±4.0 | 39.9±3.3 | 60.1±3.3 |

### [Experimental Example 3: Expression analysis of atrial myocyte markers in cell subpopulations "CD151-high" and "CD151-low" obtained under Notch inhibition]

The expression of marker genes indicating Notch signal activity and atrial myocyte markers was analyzed for the cardiomyocyte subpopulations "DMSO/CD151-high", "DMSO/CD151-low", "Notch inhibitor/CD151-high", and "Notch inhibitor/CD151-low" obtained in Experimental Example 2.

According to the conventional method, total RNA was extracted from each cell subpopulation and expression analysis was performed for the target gene of Notch signal (HEY2) and the marker genes of atrial myocytes (NR2F1, NR2F2, NPPA, KCNA5, KCNJ3, TBX5 and MYL7). The results are shown in Fig. 2. For HEY2, a relative value is shown with the expression level in Notch inhibitor/CD151-high as 1, and for the atrial myocyte marker gene group, a relative value is shown with the expression level in DMSO-added cells as 1 (mean±standard error).

The expression of the HEY2 gene, which is controlled by the Notch signal, was reduced by the addition of the Notch signal inhibitor, regardless of the expression of CD151. It is thus clear that the Notch signal was suppressed in both groups. Furthermore, the expression of atrial myocyte markers increased in both the cell subpopulations "CD151-high" and "CD151-low" obtained by adding the Notch signal inhibitor to the atrial myocyte differentiation conditions, compared to the DMSO-added control population. Therefore, it was found that the inhibition of the Notch signal promotes the expression of genes important for the differentiation of atrial myocytes.

### [Experimental Example 4: Determination of proportion of atrial myocytes in population of atrial myocytes differentiated under Notch signal inhibition]

The proportion of atrial myocytes was determined by an electrophysiological method for the "Notch inhibitor/CD151-high" and "Notch inhibitor/CD151-low" cardiomyocyte subpopulations obtained in Experimental Example 2.

Each cell subpopulation was cultured on a fibronectin-coated cover glass. Culture was performed at 37°C under normal oxygen conditions using StemPro-34 SFM supplemented with 1% L-glutamine, 150 µg/ml transferrin, 50 µg/ml ascorbic acid, 4 x 10⁻⁴ M monothioglycerol, and 5 ng/ml VEGF, with medium change every 3 days. Cells on the 15th and 16th days of culture were subjected to electrophysiological analysis.

Analysis was performed using the whole-cell patch clamp method using Axopatch 200B amplifier (Molecular Devices) and pCLAMP software. Electrodes were made with glass capillaries (WPI) using a micropipette puller and filled with intracellular solution (130 mM KOH, 130 mM L-aspartic acid, 20 mM KCl, 5 mM NaCl, 10 mM HEPES, 5 mM Mg-ATP, 10 mM EGTA, 1 mM MgCl₂, pH 7.2). Experiments were performed at 35-37°C while perfusing Gey's balanced salt solution (Sigma) as the extracellular solution. The spontaneous action potential of each cell was recorded for 1 min in current clamp mode. APD30, APD90, and maximum velocity of waveform rise (dv/dtmax) were calculated from the average waveform of 8-10 consecutive waveforms. Cells showing a waveform with APD30/90 of less than 0.3 and dv/dtmax of 10 or more were defined as atrial myocytes. Cells showing a waveform with APD30/90 of 0.3 or more and dv/dtmax of 10 or more or dv/dtmax of less than 10 were classified as other cells. The results of a comparison between the proportion of atrial myocytes in the "Notch inhibitor/CD151-high" and "Notch inhibitor/CD151-low" cardiomyocyte subpopulations obtained in Experimental Example 2 and the proportion of atrial myocytes in the "CD151-high" and "CD151-low" cardiomyocyte subpopulations obtained by culturing in cardiomyocyte induction medium 3 not containing Notch inhibitor and the solvent DMSO are shown in Table 2.

**[Table 2]**

| | CD151-high | | CD151-low | |
|---|---|---|---|---|
| | No treat (n=16) | Notch inhibitor (n=19) | No treat (n=16) | Notch inhibitor (n=20) |
| atrial myocytes (%) | 0 | 32 | 38 | 80 |
| other cells (%) | 100 | 68 | 63 | 20 |

As shown in Table 2 and Fig. 3, which shows the results of Table 2 in a bar graph, the cell subpopulation "CD151-low" obtained under conditions with the addition of a Notch signal inhibitor during atrial myocyte differentiation contained the highest proportion (80%) of atrial myocytes. In addition, the cell subpopulation "CD151-high" differentiated without the addition of a Notch signal inhibitor did not contain atrial myocytes, but obtained 32% of the cells as atrial myocytes by adding a Notch inhibitor. From the above results, it was clarified that differentiation into atrial myocytes is promoted by adding a Notch signal inhibitor during the atrial muscle differentiation process, and that atrial myocyte populations can be obtained with high efficiency by selecting atrial myocytes with CD151.

### [Industrial Applicability]

The present invention makes it possible to produce atrial myocytes from stem cells with high efficiency. In addition, the method of the present invention makes it possible to obtain a cell population predominantly containing atrial myocytes. Therefore, the present invention can contribute to the development of physiological, pharmacological, and/or toxicological analysis and screening techniques regarding atrial myocytes, aiming at the development of regenerative medicine techniques using atrial myocytes, the construction of disease pathology models using patient cells and the like, and the discovery of therapeutic drugs for cardiac diseases such as atrial fibrillation and the like.

This application is based on a patent application No. 2022-125789 filed in Japan (filing date: August 5, 2022), the contents of which are incorporated in full herein by reference.

## Claims

1. A method for producing an atrial myocyte from an early cardiomyocyte, comprising (a) a step of culturing a cell population containing early cardiomyocytes in the presence of a Notch signal inhibitor.

2. The method of claim 1, wherein the aforementioned early cardiomyocyte is derived from a pluripotent stem cell.

3. The method of claim 1, wherein the aforementioned Notch signal inhibitor is a γ secretase inhibitor.

4. The method of claim 3, wherein the aforementioned Notch signal inhibitor is at least one compound selected from the group consisting of LY411575, Deshydroxy LY-411575, DAPT, Dibenzazepine, L-685,458, RO4929097, compound E, Tarenflurbil, Avagacestat, Nirogacestat, Crenigacestat, MK0752, MRK003, BPN-15606, CB-103, RBPJ inhibitor 1, SAHM1, IMR-1A and IMR-1, as well as derivatives thereof, and salts, solvates and isomers thereof having Notch signal transduction pathway inhibition property.

5. The method of claim 1, comprising (b) a step of enriching the atrial myocyte from the aforementioned cell population by using an expression level of CD151 as an index.

6. A cell population comprising atrial myocytes, which is obtained by the method of claim 1.

7. A method for producing an atrial myocyte from a stem cell, comprising
(A) a step of inducing a cell population containing early cardiomyocytes from the aforementioned stem cells,
(B) a step of culturing the aforementioned cell population containing early cardiomyocytes in the presence of a Notch signal inhibitor, and
(C) a step of enriching the atrial myocyte from the aforementioned cell population by using an expression level of CD151 as an index.

8. A reagent for producing an atrial myocyte from an early cardiomyocyte, comprising a Notch signal inhibitor.

9. The reagent of claim 8, comprising a probe for detecting CD151.

10. Use of a Notch signal inhibitor in the production of an atrial myocyte from an early cardiomyocyte.
